(19) Europäisches Patentamt
European Patent Office
Office européen des brevets

(11) Publication number : **0 412 707 B1**

(12) # EUROPEAN PATENT SPECIFICATION

(45) Date of publication of patent specification : **09.02.94 Bulletin 94/06**

(51) Int. Cl.$^5$ : **A61K 7/06,** A61K 7/48

(21) Application number : **90308391.3**

(22) Date of filing : **31.07.90**

(54) **Hair conditioning and styling compositions.**

(30) Priority : **07.08.89 US 390559**
**06.04.90 US 505760**

(43) Date of publication of application :
**13.02.91 Bulletin 91/07**

(45) Publication of the grant of the patent :
**09.02.94 Bulletin 94/06**

(84) Designated Contracting States :
**AT BE CH DE DK ES FR GB GR IT LI LU NL SE**

(56) References cited :
**US-A- 4 136 250**
**US-A- 4 728 571**
**US-A- 4 834 972**
**CHEMICAL ABSTRACTS, vol. 88, 1978, page 271, abstract no. 54975g, Columbus, Ohio, US; & JP-A-77 57 337 (MATSUSHITA ELECTRIC WORKS, LTD) 11-05-1977**

(73) Proprietor : **THE PROCTER & GAMBLE COMPANY**
**One Procter & Gamble Plaza**
**Cincinnati Ohio 45202 (US)**
Proprietor : **MINNESOTA MINING AND MANUFACTURING COMPANY**
**3M Center, P.O. Box 33427**
**St. Paul, Minnesota 55133-3427 (US)**

(72) Inventor : **Torgerson, Peter Marte**
**4127 U.S. Rt. 35 NW**
**Washington Court House OH 43160 (US)**
Inventor : **Bolich, Raymond Edward, Jr.**
**7201 Striker Rd.**
**Maineville, OH 45039 (US)**
Inventor : **Garbe, James Edward**
**4890 Ashley Le. Ap. 201**
**Inver Grove Heights, MN 55075 (US)**

(74) Representative : **Brooks, Maxim Courtney et al**
**Procter & Gamble (NTC) Limited Whitley Road**
**Longbenton**
**Newcastle-upon-Tyne NE12 9TS (GB)**



Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

The present invention relates to hair care compositions which have improved hair conditioning and style retention properties while still leaving the hair with a natural non-sticky feel. These compositions utilize a group of specific silicone macromer-containing copolymers.

The desire to have hair retain a particular shape is widely held. Such style retention is generally accomplished by either of two routes: permanent chemical alteration or temporary alteration of hair style/shape. A temporary alteration is one which can be removed by water or by shampooing. Temporary style alteration has generally been accomplished by means of the application of a composition to dampened hair after shampooing and/or conditioning and prior to drying and/or styling. The materials used to provide setting benefits have generally been resins or gums and have been applied in the form of mousses, gels, lotions, or sprays. This approach presents several significant drawbacks to the user. It requires a separate step following shampooing/conditioning to apply the styling composition. In addition, since the style hold is provided by resin materials which set-up on the hair, the hair tends to feel sticky or stiff after application and it is difficult to restyle the hair without further application of the styling composition.

It has now been discovered that hair care compositions comprising certain specifically-defined silicone macromer-containing copolymers provide excellent hair style retention benefits, together with hair conditioning. The compositions may be in any of the conventional forms including, but not limited to, shampoos, conditioners, hair sprays, tonics, lotions, gels, and mousses. The compositions provide these benefits to hair without leaving the hair with a stiff or sticky/tacky feel and without negatively affecting dry hair properties, such as ease of combing. Further, hair to which the compositions of the present invention have been applied may be restyled several times without requiring reapplication of the compositions.

These results are surprising since other materials which have been typically used in hair care compositions to provide style retention, such as resins and gums, generally hurt dry hair properties (e.g., combing) and leave hair with a sticky and/or stiff feel. Furthermore, silicone materials typically used for hair conditioning tend to hurt style retention.

Siloxanes (see, for example, US-A-3,208,911, Oppliger, issued September 28, 1965) and siloxane-containing polymers have been taught for use in hair conditioning compositions. US-A-4,601,902, Fridd et al., issued July 22, 1986, describes hair conditioning or shampoo/conditioner compositions which include a polydiorganosiloxane having quaternary ammonium substituted groups attached to the silicon, and a polydiorganosiloxane having silicon-bonded substituents which are amino-substituted hydrocarbon groups. US-A-4,654,161, Kollmeier et al., issued March 31, 1987, describes a group of organopolysiloxanes containing betaine substituents. When used in hair care compositions, these compounds are said to provide good conditioning, compatibility with anionic components, hair substantivity, and low skin irritation. US-A-4,563,347, Starch, issued January 7, 1986, relates to hair conditioning compositions which include siloxane components containing substituents to provide attachment to hair. JP-A-56-129,300, Lion Corporation, published October 9, 1981, relates to shampoo conditioner compositions which include an organopolysiloxane-oxyalkylene copolymer together with an acrylic resin. US-A-4,479,893, Hirota et al., issued October 30, 1984, describes shampoo conditioner compositions containing a phosphate ester surfactant and a silicon derivative (e.g., polyether- or alcohol-modified siloxanes). JP-A-52-57337, describes hair conditioning lotions which include a copolymer of N-vinyl pyrrolidone and tris(trimethylsiloxy)vinylsilane. Polyether-modified polysiloxanes are also disclosed for use in shampoo in US-A-3,957,970, Korkis, issued May 18, 1976. US-A-4,185,087, Morlino issued January 22, 1980, describes quaternary nitrogen derivatives of trialkylamino hydroxy organosilicon compounds which are said to have superior hair conditioning properties.

Siloxane-derived materials have also been used in hair styling compositions. JP-A-56-092,811, Lion Corporation, published December 27, 1979, describes hair setting compositions which comprise an amphoteric acrylic resin, a polyoxyalkylene-denatured organopolysiloxane, and polyethylene glycol. US-A-4,744,978, Homan et al., issued May 17, 1988, describes hair styling compositions (such as hair sprays) which include the combination of a carboxyfunctional polydimethylsiloxane and a cationic organic polymer containing amine or ammonium groups. Hair styling compositions which include polydiorganosiloxanes and a cationic organic polymer are taught in US-A-4,733,677, Gee et al., issued March 29, 1988, and US-A-4,724,851, Cornwall et al., issued February 16, 1988. Finally, EP-A-117,360, Cantrell et al., published September 5, 1984, discloses compositions, containing a siloxane polymer having at least one nitrogen-hydrogen bond, a surfactant, and a solubilized titanate, zirconate or germanate, which act as both a conditioner and a hair styling aid.

Siloxane-containing polymers have also been used in non-hair care applications. US-A-4,136,250, Mueller et al., issued January 23, 1979, relates to polymeric materials used in biological contexts where oxygen permeable and tissue compatible membranes are required. They can also be used as carriers for biologically-active substances. These materials are hydrophilic water-insoluble gels which include a low molecular weight terminal olefinic siloxane macromer and a polymer containing water-soluble monoolefinic monomer. US-A-4,693,935, Mazurek, issued September 15, 1987, describes pressure sensitive adhesive compositions which include a copolymer with a vinyl polymeric backbone having grafted thereto polysiloxane moieties. US-A-4,728,571, Clemens et al., issued March 1, 1988, relates to adhesive release coating compositions which comprise polysiloxane-grafted copolymers and blends of those copolymers with other polymeric materials. None of these last three patents suggest the use of the disclosed siloxane-containing polymers in hair care compositions.

It is an object of the present invention to formulate hair care compositions which provide effective hair conditioning and style retention properties.

It is also an object of the present invention to formulate hair care compositions which provide conditioning and style retention from a single composition.

It is a further object of the present invention to formulate hair care compositions which provide good style retention without leaving hair with a stiff or sticky/tacky feel.

It is a further object of the present invention to provide an improved method for styling and conditioning hair.

These and other objects will become readily apparent from the detailed description which follows.

Unless otherwise indicated, all percentages and ratios herein are by weight.

According to the present invention there is provided a hair care composition comprising:

(a) from 0.1% by weight to 10.0% by weight of a silicone-containing copolymer having a molecular weight of from 10,000 to 1,000,000 comprising a component selected from: a lipophilic low polarity free radically polymerizable vinyl monomer (A), a hydrophilic polar monomer which is copolymerizable with A (B), and mixtures thereof; together with a silicone-containing macromer (C) having a weight average molecular weight of from 1,000 to 50,000 based on polydimethylsiloxane selected from

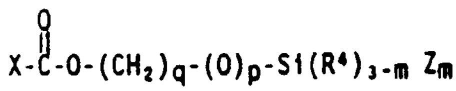

$X\text{-}Si(R^4)_{3-m}\,Z_m$

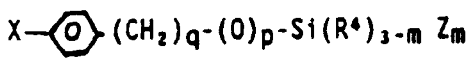

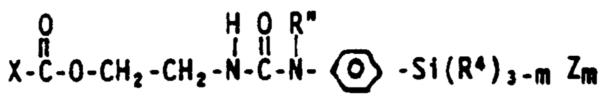

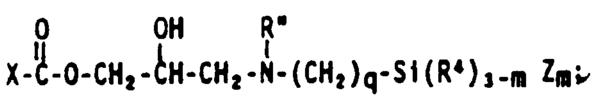

; and

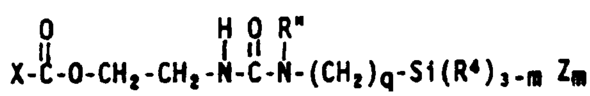

wherein m is 1, 2 or 3; p is 0 or 1; R" is alkyl or hydrogen; q is an integer from 2 to 6; s is an integer from 0 to 2; X is

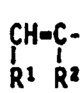

;

$R^1$ is hydrogen or -COOH; $R^2$ is hydrogen, methyl or $-CH_2COOH$; Z is

$$R^4-(-\underset{CH_3}{\overset{CH_3}{|}{Si}}-O-)_r\ ;$$

$R^4$ is alkyl, alkoxy, alkylamino, aryl or hydroxyl; and r is an integer from 5 to 700;

and wherein the silicone-containing copolymer comprises from 0% to 98% by weight monomer A, from 0% to 98% by weight monomer B, and from 0.1% by weight to 50% by weight monomer C; and

(b) from 0,5% by weight to 99.5% by weight of a carrier suitable for application to hair.

The essential, as well as the optional, components of the present invention are described below.

Silicone-Containing Copolymer

The compositions of the present invention contain from 0.1% to 10.0%, preferably from 0.5% to 8.0%, of specifically-defined silicone-containing copolymers. It is these polymers which provide the unique hair conditioning and hair setting characteristics of the present invention. The polymers have a weight average molecular weight of from 10,000 to 1,000,000 (preferably from 30,000 to 300,000) and, preferably, have a Tg of at least -20°C. As used herein, the abbreviation "Tg" refers to the glass transition temperature of the non-silicone backbone, and the abbreviation "Tm" refers to the crystalline melting point of the non-silicone backbone, if such a transition exists for a given polymer.

Preferred polymers comprise a vinyl polymeric backbone, preferably having a Tg above -20°C and, grafted to the backbone, a polydimethylsiloxane macromer having a weight average molecular weight of from 1,000 to 50,000, preferably from 5,000 to 40,000, most preferably 20,000. The polymer is such that when it is formulated into the finished hair care composition, when dried, the polymer phase separates into a discontinuous phase which includes the polydimethylsiloxane macromer and a continuous phase which includes the backbone. It is believed that this phase separation property provides a specific orientation of the polymer on hair which results in the desired hair conditioning and setting benefits. The phase-separating nature of the compositions of the present invention may be determined as follows:

The polymer is cast as a solid film out of a good solvent (i.e., a solvent which dissolves both the backbone and the silicone). This film is then sectioned and examined by transmission electron micrography. Microphase separation is demonstrated by the observation of inclusions in the continuous phase. These inclusions should have the proper size to match the size of the silicone chain (typically a few hundred nm or less) and the proper density to match the amount of silicone present. This behavior is well documented in the literature for polymers with this structure (see, for example, S. D. Smith, Ph.D. Thesis, University of Virginia, 1987, and references cited therein).

A second method for determining phase-separating characteristics involves examining the enrichment of the concentration of silicone at the surface of a polymer film relative to the concentration in the bulk polymer. Since the silicone prefers the low energy air interface, it preferentially orients on the polymer surface. This produces a surface which is entirely covered by silicone even when the concentration of the silicone by weight in the whole polymer is low (2% to 20%). This is demonstrated experimentally by ESCA (electron spectroscopy for chemical analysis) of the dried film surface. Such an analysis shows a high level of silicone and a greatly reduced level of backbone polymer when the film surface is analyzed. (Surface here means the first few tens of Angstroms of film thickness.) By varying the angle of the interrogating beam the surface can be analyzed to varying depths.

In its broadest aspect, the copolymers utilized in the present application comprise C monomers together with monomers selected from the group consisting of A monomers, B monomers, and mixtures thereof. These copolymers contain at least A or B monomers, together with C monomers, and preferred copolymers contain A, B and C monomers.

Examples of useful copolymers and how they are made are described in detail in US-A-4,696,935, Mazurek, issued September 15, 1987, and US-A-4,728,571, Clemens et al., issued March 1, 1988. These copolymers are comprised of monomers A, C and, optionally, B, which are defined as follows. A is at least one free-radically polymerizable vinyl monomer or monomers. B, when used, comprises at least one reinforcing monomer copolymerizable with A and is selected from the group consisting of polar monomers and macromers having a Tg or a Tm above -20°C. When used, B is up to 98%, preferably up to 80%, more preferably up to 20%, of the total monomers in the copolymer. Monomer C comprises from 0.01% to 50.0% of the total monomers in the copolymer.

Representative examples of A (hydrophobic) monomers are the acrylic or methacrylic acid esters of $C_1$-

$C_{18}$ alcohols, such as methanol, ethanol, methoxy ethanol, 1-propanol, 2-propanol, 1-butanol, 2-methyl-1-propanol, 1-pentanol, 2-pentanol, 3-pentanol, 2-methyl-1-butanol, 1-methyl-1-butanol, 3-methyl-1-butanol, 1-methyl-1-pentanol, 2-methyl-1-pentanol, 3-methyl-1-pentanol, t-butanol(2-methyl-2-propanol), cyclohexanol, neodecanol, 2-ethyl-1-butanol, 3-heptanol, benzyl alcohol, 2-octanol, 6-methyl-1-heptanol, 2-ethyl-1-hexanol, 3,5-dimethyl-1-hexanol, 3,5,5-tri-methyl-1-hexanol, 1-decanol, 1-dodecanol, 1-hexadecanol, and 1-octadecanol, the alcohols having from 1-18 carbon atoms with the average number of carbon atoms being from 4-12; styrene; polystyrene macromer; vinyl acetate; vinyl chloride; vinylidene chloride; vinyl propionate; alpha-methylstyrene; t-butylstyrene; butadiene; cyclohexadiene; ethylene; propylene; vinyl toluene; and mixtures thereof. Preferred A monomers include n-butyl methacrylate, isobutyl methacrylate, 2-ethylhexyl methacrylate, methyl methacrylate, t-butylacrylate, t-butylmethacrylate, and mixtures thereof.

Representative examples of B (hydrophilic) monomers include acrylic acid, methacrylic acid, N,N-dimethylacrylamide, dimethylaminoethyl methacrylate, quaternized dimethylaminoethyl methacrylate, methacrylamide, N-t-butylacrylamide, maleic acid, maleic anhydride and its half esters, crotonic acid, itaconic acid, acrylamide, acrylate alcohols, hydroxyethyl methacrylate, diallyldimethyl ammonium chloride, vinyl pyrrolidone, vinyl ethers (such as methyl vinyl ether), maleimides, vinyl pyridine, vinyl imidazole, other polar vinyl heterocyclics, styrene sulfonate, allyl alcohol, vinyl alcohol (produced by the hydrolysis of vinyl acetate after polymerization) vinyl caprolactam, and mixtures thereof. Preferred B monomers include acrylic acid, N,N-dimethylacrylamide, dimethylaminoethyl methacrylate, quaternized dimethylaminoethyl methacrylate, vinyl pyrrolidone, and mixtures thereof.

C has a weight average molecular weight of from 1,000 to 50,000, preferably from 5,000 to 40,000, most preferably from 10,000 to 20,000. The C monomer has a formula selected from the following group:

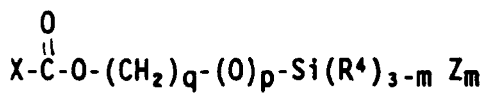

(a preferred monomer, particularly preferred when p = 0 and q = 3)

$$X\text{-}Si(R^4)_{3-m}\ Z_m$$

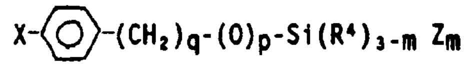

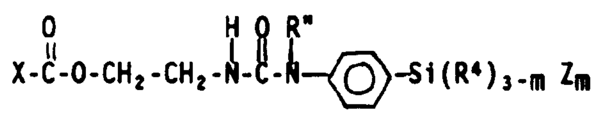

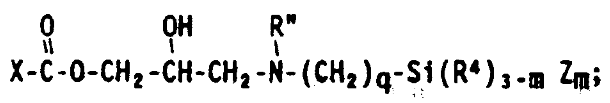 and

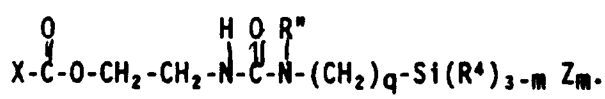

In those structures, m is 1, 2 or 3 (preferably m = 1); p is 0 or 1; R" is alkyl or hydrogen; q is an integer from 2 to 6; s is an integer from 0 to 2; X is

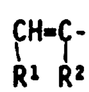 ;

$R^1$ is hydrogen or -COOH (preferably $R^1$ is hydrogen); $R^2$ is hydrogen, methyl or $-CH_2COOH$ (preferably $R^2$ is methyl); Z is

$$R^4-(-\overset{CH_3}{\underset{CH_3}{\overset{|}{\underset{|}{Si}}}}-O-)_r;$$

$R^4$ is alkyl, alkoxy, alkylamino, aryl, or hydroxyl (preferably $R^4$ is alkyl); and r is an integer from 5 to 700 (preferably r is about 250).

The polymers of the present invention comprise from 0% to 98% (preferably from 5% to 98%, more preferably from 50% to 90%) of monomer A, from 0% to 98% (preferably from 7.5% to 80%) of monomer B, and from 0.1% to 50% (preferably from 0.5% to 40%, most preferably from 2% to 25%) of monomer C. The combination of the A and B monomers preferably comprises from 50.0% to 99.9% (more preferably 60% to 99%, most preferably from 75% to 95%) of the polymer. The composition of any particular copolymer will help determine its formulational properties. In fact, by appropriate selection and combination of particular A, B and C components, the copolymer can be optimized for inclusion in specific vehicles. For example, polymers which are soluble in an aqueous formulation preferably have the composition: from 0% to 70% (preferably from 5% to 70% monomer A, from 30% to 98% (preferably from 30% to 80% monomer B, and from 1% to 40% monomer C. Polymers which are dispersible have the preferred composition: from 0% to 70% (more preferably from 5% to 70%) monomer A, from 20% to 80% (more preferably from 20% to 60%) monomer B, and from 1% to 40% monomer C.

In one aspect of the present invention, the polymers comprise from 5% to 98% A monomer, from 0.01% to 50% C monomer, and from 0% to 98% B monomer. In these polymers, it is preferred that A be selected from t-butylacrylate, t-butylmethacrylate, and mixtures thereof, since such polymers can be dissolved directly in cyclomethicone solvents without requiring co-solvents. This is surprising in view of US-A-4,693,935 (Mazurek) and US-A-4,728,571 (Clemens et al.) which suggest that tertiary alcohols are not suitable A monomers.

Particularly preferred polymers for use in the present invention include the following (the weight percents below refer to the amount of reactants added in the polymerization reaction, not necessarily the amount in the finished polymer):

acrylic acid/n-butylmethacrylate/(polydimethylsiloxane (PDMS) macromer-20,000 molecular weight) (10/70/20 w/w/w) (I)

N,N-dimethylacrylamide/isobutyl methacrylate/(PDMS macromer - 20,000 molecular weight) (20/60/20 w/w/w) (II)

dimethylaminoethyl methacrylate/isobutyl methacrylate/2-ethylhexylmethacrylate/(PDMS macromer-20,000 molecular eight) (25/40/15/20 w/w/w/w) (III)

dimethylacrylamide/(PDMS macromer - 20,000 molecular weight) (80/20 w/w) (IV)

t-butylacrylate/t-butylmethacrylate/(PDMS macromer - 10,000 molecular weight) (56/24/20 w/w/w) (V)

t-butylacrylate/(PDMS macromer - 10,000 molecular weight) (80/20 w/w) (VI)

t-butylacrylate/N,N-dimethylacrylamide/(PDMS macromer 10,000 molecular weight) (70/10/20) (VII)

t-butylacrylate/acrylic acid/(PDMS macromer - 10,000 molecular weight) (75/5/20) (VIII)

The silicone-containing copolymers described above are synthesized as follows.

The polymers are synthesized by free radical polymerization methods, the general principles of which are well understood. See, for example, Odian, "Principles of Polymerization", 2nd edition, John Wiley & Sons, 1981, pp. 179-318. The desired monomers are all placed in a reactor, along with a sufficient amount of a mutual solvent so that when the reaction is complete the viscosity of the reaction is reasonable. Typical monomer loadings are from 20% to 50%. Undesired terminators, especially oxygen, are removed as needed. This is done by evacuation or by purging with an inert gas, such as argon or nitrogen. The initiator is introduced and the reaction brought to the temperature needed for initiation to occur, assuming thermal initiators are used. Alternatively, redox or radiation initiation can be used as desired. The polymerization is allowed to proceed as long as needed for a high level of conversion to be achieved, typically from a few hours to a few days. The solvent is then removed, usually by evaporation or by precipitating the polymer by addition of a nonsolvent. The polymer is further purified as needed.

By way of example, Polymers I, II and III, described above, are synthesized in the following manner. There are numerous variations on these procedures which are entirely up to the discretion of the synthetic chemist (e.g., choice of degassing method and gas, choice of initiator type, extent of conversion, reaction loading, etc). The choice of initiator and solvent are often determined by the requirements of the particular monomers used, since different monomers have different solubilities and different reactivities to a specific initiator.

Polymer I: Place 10 parts acrylic acid, 70 parts n-butyl-methacrylate, and 20 parts 20K PDMS macromer in a flask. Add sufficient ethyl acetate to produce a final monomer concentration of 40%. Add initiator, benzoyl

peroxide, to a level of 0.5% by weight relative to the amount of monomer. Evacuate the vessel, and refill with nitrogen. Heat to 60°C and maintain this temperature for 48 hours while agitating. Terminate the reaction by cooling to room temperature, and dry off the ethyl acetate by pouring the reaction mixture into a teflon (RTM) coated pan and placing in a vacuum oven.

Polymer II: Place 20 parts N,N-dimethylacrylamide, 60 parts isobutylmethacrylate, and 20 parts silicone macromer in a reaction vessel fitted with a temperature probe, reflux condenser, inlet port, and argon sparge. Add sufficient toluene to bring the final monomer concentration to 20% by weight. Sparge with argon for 1 to 2 hours. While sparging, heat to 62°C, with a sufficient rate of argon flow to keep the solution mixed. Add initiator, azobisisobutyronitrile, to a level of 0.25% by weight relative to the weight of monomer present. Monitor the reaction visually, ensuring that no phase separation of reactants occurs during polymerization. If any turbidity is observed, add sufficient warm degassed toluene to eliminate the turbidity. Continue to monitor throughout the reaction. Terminate the reaction after 4 to 6 hours and purify as with Polymer I.

Polymer III: Place 25 parts dimethylaminoethylmethacrylate, 15 parts 2-ethylhexylmethacrylate, 40 parts isobutylmethacrylate, and 20 parts 20K PDMS macromer in a reaction vessel fitted with a mechanical stirrer, argon sparge, temperature probe, reflux condenser and inlet port. Add sufficient toluene to bring the final monomer concentration to 30% by weight. Begin stirring and sparge with argon for 2 hours. While sparging, heat to 60°C in a water bath. Add initiator, azobisisobutyronitrile, to a level of 0.15% by weight relative to the weight of monomer present. Continue stirring and a slow argon sparge and maintain the reaction temperature at 60°C. Allow to react for 24 hours. Terminate the reaction and remove the solvent as with Polymer I.

## Carrier

The compositions of the invention also comprise a carrier, or a mixture of such carriers, which are suitable for application to hair. The carriers are present at from 0.5% to 99.5%, preferably from 5.0% to 99.5%, most preferably from 10.0% to 90.0%, of the composition. As used herein, the phrase "suitable for application to hair" means that the carrier does not damage or negatively affect the aesthetics of hair or cause irritation to skin. Choice of appropriate carrier will also depend on the particular copolymer to be used, and whether the product formulated is meant to be left on hair (e.g., hair spray, mousse, tonic) or rinsed off (e.g., shampoo, conditioner) after use.

The carriers used herein include solvents, as well as other carrier or vehicle components conventionally used in hair care compositions. The solvent selected must be able to dissolve or disperse the particular silicone copolymer being used. The nature and proportion of B monomer in the copolymer largely determines its polarity and solubility characteristics. The silicone copolymers can be designed, by appropriate combination of monomers, for formulation with a wide range of solvents. Suitable solvents for use in the present invention include, but are not limited to, water, lower alcohols (such as ethanol, isopropanol), hydroalcoholic mixtures, hydrocarbons (such as isobutane, hexane, decene, acetone), halogenated hydrocarbons (such as Freon RTM), linalool, hydrocarbon esters (such as ethyl acetate, dibutyl phthalate), volatile silicon derivatives, especially siloxanes (such as phenyl pentamethyl disiloxane, methoxypropyl heptamethyl cyclotetrasiloxane, chloropropyl pentamethyl disiloxane, hydroxypropyl pentamethyl disiloxane, octamethyl cyclotetrasiloxane, decamethyl cyclopentasiloxane), and mixtures thereof. Preferred solvents include water, ethanol, volatile silicon derivatives, and mixtures thereof. The solvents used in such mixtures may be miscible or immiscible with each other.

Where the hair care compositions are conditioner compositions, the carrier may include gel vehicle materials. This gel vehicle comprises two essential components: a lipid vehicle material and a cationic surfactant vehicle material. Cationic surfactant materials are described in detail below. Gel-type vehicles are generally described in the following documents, Barry, "The Self Bodying Action of the Mixed Emulsifier Sodium Dodecyl Sulfate/Cetyl Alcohol", 28 J. of Colloid and Interface Science 82-91 (1968); Barry, et al., "The Self-Bodying Action of Alkyltrimethylammonium Bromides/Cetostearyl Alcohol Mixed Emulsifiers; Influence of Quaternary Chain Length", 35 J. of Colloid and Interface Science 689-708 (1971); and Barry, et al., "Rheology of Systems Containing Cetomacrogol 1000 - Cetostearyl Alcohol, I. Self Bodying Action", 38 J. of Colloid and Interface Science 616-625 (1972).

The vehicles may incorporate one or more lipid vehicle materials which are essentially water-insoluble, and contain hydrophobic and hydrophilic moieties. Lipid vehicle materials include naturally or synthetically-derived acids, acid derivatives, alcohols, esters, ethers, ketones, and amides with carbon chains of from 12 to 22, preferably from 16 to 18, carbon atoms in length. Fatty alcohols and fatty esters are preferred; fatty alcohols are particularly preferred.

Lipid vehicle materials among those useful herein are disclosed in Bailey's Industrial Oil and Fat Products, (3rd edition, D. Swern, ed., 1979), Fatty alcohols included among those useful herein are disclosed in the following documents, US-A-3,155,591, Hilfer, issued November 3, 1964; US-A-4,165,369, Watanabe, et al., issued August 21, 1979; US-A-4,269,824, Villamarin, et al., issued May 26, 1981; BP-A 1,532,585, published November 15, 1978; and Fukushima, et al., "The Effect of Cetostearyl Alcohol in Cosmetic Emulsions", 98 Cosmetics & Toiletries 89-112 (1983). Fatty esters included among those useful herein are disclosed in US-A 3,341,465, Kaufman, et al., issued September 12, 1976. If included in the compositions of the present invention, the lipid vehicle material is present at from 0.1% to 10.0% of the composition; the cationic surfactant vehicle material is present at from 0.5% to 5.0% of the composition.

Preferred esters for use herein include cetyl palmitate and glycerylmonostearate. Cetyl alcohol and stearyl alcohol are preferred alcohols. A particularly preferred lipid vehicle material is comprised of a mixture of cetyl alcohol and stearyl alcohol containing from 55% to 65% (by weight of mixture) of cetyl alcohol.

Preferred vehicles for use in the compositions of the present invention include combinations of hydrophobically-modified hydroxyethyl cellulose materials with thickeners (such as locust bean gum), particular surfactants, quaternary ammonium compounds (such as ditallowdimethyl ammonium chloride), and/or chelating agents (such as EDTA).

Other carriers, suitable for use with the present invention are, for example, those used in the formulation of tonics, mousses, gels and hair sprays. Tonics, gels and non-aerosol hair sprays utilize a solvent such as water or alcohol while mousses and aerosol hair sprays additionally utilize a propellant such as trichlorofluoromethane, dichlorodifluoromethane, dimethylether, propane, n-butane or isobutane. A tonic or hair spray product having a low viscosity may also require an emulsifying agent to keep the silicone copolymer homogeneously dispersed in solution. Examples of suitable emulsifying agents include nonionic, cationic, anionic surfactants, or mixtures thereof. If such an emulsifying agent is used, it is present at a level of from 0.25% to 7.5% of the composition. The level of propellant can be adjusted as desired but is generally from 3% to 30% of mousse compositions and from 15% to 50% of the aerosol hair spray compositions.

## Optional Ingredients

The hair care compositions of the present invention may be formulated in a wide variety of product types, including mousses, gels, lotions, tonics, sprays, shampoos and conditioners. The additional components required to formulate such products vary with product type and can be routinely chosen by one skilled in the hair care product art. The following is a description of some of these additional components.

### Surfactants

Surfactants are preferred optional ingredients in the compositions of the invention, particularly shampoo and conditioner compositions. When present, the surfactant comprises from 0.05% to 50% of the composition. For a shampoo, the level is preferably from 10% to 30%, most preferably from 12% to 25%, of the composition. For conditioners, the preferred level of surfactant is from 0.2% to 3%. Surfactants useful in compositions of the present invention include anionic, nonionic, cationic, zwitterionic and amphoteric surfactants.

Synthetic anionic detergents useful herein, particularly for shampoo compositions, include alkyl and alkyl ether sulfates. These materials have the respective formulae $ROSO_3M$ and $RO(C_2H_4O)_xSO_3M$, wherein R is alkyl or alkenyl of from 10 to 20 carbon atoms, x is 1 to 10, and M is a water-soluble cation such as ammonium, sodium, potassium and triethanolamine. The alkyl ether sulfates useful in the present invention are condensation products of ethylene oxide and monohydric alcohols having from 10 to 20 carbon atoms. Preferably, R has from 12 to 18 carbon atoms in both the alkyl and alkyl ether sulfates. The alcohols can be derived from fats, e.g., coconut oil or tallow, or can be synthetic. Lauryl alcohol and straight chain alcohols derived from coconut oil are preferred herein. Such alcohols are reacted with 1 to 10, and especially 3, molar proportions of ethylene oxide and the resulting mixture of molecular species, having, for example, an average of 3 moles of ethylene oxide per mole of alcohol, is sulfated and neutralized.

Specific examples of alkyl ether sulfates which may be used in the present invention are sodium coconut alkyl triethylene glycol ether sulfate; sodium tallow alkyl triethylene glycol ether sulfate; and sodium tallow alkyl hexaoxyethylene sulfate. Highly preferred alkyl ether sulfates are those comprising a mixture of individual compounds, said mixture having an average alkyl chain length of from 12 to 16 carbon atoms and an average degree of ethoxylation of from 1 to 4 moles of ethylene oxide. Such a mixture also comprises from 0 to 20% by weight $C_{12-13}$ compounds; from 60 to 100% by weight of $C_{14-15-16}$ compounds, from 0 to 20% by weight of $C_{17-18-19}$ compounds; from 3 to 30% by weight of compounds having a degree of ethoxylation of 0; from 45 to 90% by weight of compounds having a degree of ethoxylation of from 1 to 4; from 10 to 25% by weight of compounds having a degree of ethoxylation of from 4 to 8; and from 0.1 to 15% by weight of compounds having a degree of ethoxylation greater than 8.

Another suitable class of anionic surfactants are the water-soluble salts of the organic, sulfuric acid reaction products of the general formula:

$$R_1\text{--}SO_3\text{--}M$$

wherein $R_1$ is chosen from the group consisting of a straight or branched chain, saturated aliphatic hydrocarbon radical having from 8 to 24, preferably 12 to 18, carbon atoms; and M is a cation. Important examples are the salts of an organic sulfuric acid reaction product of a hydrocarbon of the methane series, including iso-, neo-, ineso-, and n-paraffins, having 8 to 24 carbon atoms, preferably 12 to 18 carbon atoms and a sulfonating agent, e.g., $SO_3$, $H_2SO_4$, oleum, obtained according to known sulfonation methods, including bleaching and hydrolysis. Preferred are alkali metal and ammonium sulfonated $C_{12-18}$ n-paraffins.

Additional examples of anionic synthetic surfactants which come within the terms of the present invention are the reaction products of fatty acids esterified with isethionic acid and neutralized with sodium hydroxide where, for example, the fatty acids are derived from coconut oil; sodium or potassium salts of fatty acid amides of methyl tauride in which the fatty acids, for example, are derived from coconut oil. Other anionic synthetic surfactants of this variety are set forth in US-A 2,486,921; US-A 2,486,922; and US-A 2,396,278.

Still other anionic synthetic surfactants include the class designated as succinamates. This class includes such surface active agents as disodium N-octadecylsulfosuccinamate; tetrasodium N-(1,2-dicarboxyethy)-N-octadecylsulfosuccinamate; diamyl ester of sodium sulfosuccinic acid; dihexyl ester of sodium sulfosuccinic acid; dioctyl esters of sodium sulfosuccinic acid.

Other suitable anionic surfactants utilizable herein are olefin sulfonates having 12 to 24 carbon atoms. The term "olefin sulfonates" is used herein to mean compounds which can be produced by the sulfonation of α-olefins by means of uncomplexed sulfur trioxide, followed by neutralization of the acid reaction mixture in conditions such that any sultones which have been formed in the reaction are hydrolyzed to give the corresponding hydroxy-alkanesulfonates. The sulfur trioxide can be liquid or gaseous, and is usually, but not necessarily, diluted by inert diluents, for example by liquid $SO_2$, chlorinated hydrocarbons, etc., when used in the liquid form, or by air, nitrogen, gaseous $SO_2$, etc., when used in the gaseous form.

The α-olefins from which the olefin sulfonates are derived are mono-olefins having 12 to 24 carbon atoms, preferably 14 to 16 carbon atoms. Preferably, they are straight chain olefins. Examples of suitable 1-olefins include 1-dodecene; 1-tetradecene; 1-hexadecene; 1-octadecene; 1-eicosene and 1-tetracosene.

In addition to the true alkene sulfonates and a proportion of hydroxy-alkanesulfonates, the olefin sulfonates can contain minor amounts of other materials, such as alkene disulfonates depending upon the reaction conditions, proportion of reactants, the nature of the starting olefins and impurities in the olefin stock and side reactions during the sulfonation process.

A specific α-olefin sulfonate mixture of the above type is described more fully in the US-A-3,332,880, Pflaumer and Kessler, issued July 25, 1967,.

Another class of anionic organic surfactants are the β-alkyloxy alkane sulfonates. These compounds have the following formula:

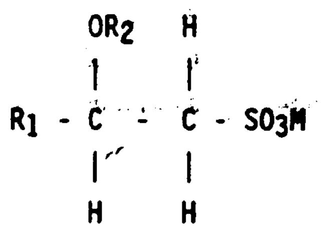

where $R_1$ is a straight chain alkyl group having from 6 to 20 carbon atoms, $R_2$ is a lower alkyl group having from 1 (preferred) to 3 carbon atoms, and M is a water-soluble cation as hereinbefore described.

Specific examples of β-alkyloxy-alkane-1-sulfonates, or alternatively 2-alkyloxy-alkane-1-sulfonates, having low hardness (calcium ion) sensitivity useful herein include: potassium-β-methoxydecanesulfonate, sodium 2-methoxy-tridecanesulfonate, potassium 2-ethoxytetradecylsulfonate, sodium 2-isopropoxyhexadecylsulfonate, lithium 2-t-butoxytetradecylsulfonate, sodium β-methoxyoctadecylsulfonate, and ammonium β-n-propoxydodecylsulfonate.

Many additional nonsoap synthetic anionic surfactants are described in McCutcheon's Detergents and Emulsifiers, 1984, Annual, published by Allured Publishing Corporation. Also US-A-3,929,678, Laughlin et al., issued December 30, 1975, discloses many other anionic as well as other surfactant types.

Nonionic surfactants, which are preferably used in combination with an anionic, amphoteric or zwitterionic surfactant, can be broadly defined as compounds produced by the condensation of alkylene oxide groups (hydrophilic in nature) with an organic hydrophobic compound, which may be aliphatic or alkyl aromatic in nature. Examples of preferred classes of nonionic surfactants are:

1. The polyethylene oxide condensates of alkyl phenols, e.g., the condensation products of alkyl phenols having an alkyl group containing from 6 to 12 carbon atoms in either a straight chain or branched chain configuration, with ethylene oxide, the said ethylene oxide being present in amounts equal to from 10 to 60 moles of ethylene oxide per mole of alkyl phenol. The alkyl substituent in such compounds may be derived from polymerized propylene, diisobutylene, octane, or nonane, for example.

2. Those derived from the condensation of ethylene oxide with the product resulting from the reaction of propylene oxide and ethylene diamine products which may be varied in composition depending upon the balance between the hydrophobic and hydrophilic elements which is desired. For example, compounds containing from 40% to 80% polyoxyethylene by weight and having a molecular weight of from 5,000 to 11,000 resulting from the reaction of ethylene oxide groups with a hydrophobic base constituted of the reaction product of ethylene diamine and excess propylene oxide, said base having a molecular weight of the order of 2,500 to 3,000, are satisfactory.

3. The condensation product of aliphatic alcohols having from 8 to 18 carbon atoms, in either straight chain or branched chain configuration, with ethylene oxide, e.g., a coconut alcohol ethylene oxide condensate having from 10 to 30 moles of ethylene oxide per mole of coconut alcohol, the coconut alcohol fraction having from 10 to 14 carbon atoms.

4. Long chain tertiary amine oxides corresponding to the following general formula:

$$R_1R_2R_3N \longrightarrow O$$

wherein $R_1$ contains an alkyl, alkenyl or monohydroxy alkyl radical of from 8 to about 18 carbon atoms, from 0 to 10 ethylene oxide moieties, and from 0 to 1 glyceryl moiety, and $R_2$ and $R_3$ contain from 1 to 3 carbon atoms and from 0 to 1 hydroxy group, e.g., methyl, ethyl, propyl, hydroxyethyl, or hydroxypropyl radicals. The arrow in the formula is a conventional representation of a semipolar bond. Examples of amine oxides suitable for use in this invention include dimethyl-dodecylamine oxide, oleyldi(2-hydroxyethyl) amine oxide, dimethyloctylamine oxide, dimethyl-decylamine oxide, dimethyl-tetradecylamine oxide, 3,6,9-trioxaheptadecyldiethylamine oxide, di(2-hydroxyethyl)-tetradecylamine oxide, 2-dodecoxyethyl-dimethylamine oxide, 3-dodecoxy-2-hydroxypropyldi(3-hydroxypropyl)amine oxide, dimethylhexadecylamine oxide.

5. Long chain tertiary phosphine oxides corresponding to the following general formula:

$$RR'R''P \longrightarrow O$$

wherein R contains an alkyl, alkenyl or monohydroxyalkyl radical ranging from 8 to 18 carbon atoms in chain length, from 0 to 10 ethylene oxide moieties and from 0 to 1 glyceryl moiety and R' and R" are each alkyl or monohydroxyalkyl groups containing from 1 to 3 carbon atoms. The arrow in the formula is a conventional representation of a semipolar bond. Examples of suitable phosphine oxides are: dodecyldimethylphosphine oxide, tetradecyldimethylphosphine oxide, tetradecylmethylethylphosphine oxide, 3,6,9,-trioxaoctadecyldimethylphosphine oxide, cetyldimethylphosphine oxide, 3-dodecoxy-2-hydroxypropyldi(2-hydroxyethyl) phosphine oxide, stearyldimethylphosphine oxide, cetylethylpropylphosphine oxide, oleyldiethylphosphlne oxide, dodecyldiethylphosphine oxide, tetradecyldiethylphosphine oxide, dodecyldipropylphosphine oxide, dodecyldi(hydroxymethyl)phosphine oxide, dodecyldi(2-hydroxyethyl)phosphine oxide, tetradecylmethyl-2-hydroxypropylphosphine oxide, oleydimethylphosphine oxide, 2-hydroxydodecyldimethylphosphine oxide.

6. Long chain dialkyl sulfoxides containing one short chain alkyl or hydroxy alkyl radical of from 1 to 3 carbon atoms (usually methyl) and one long hydrophobic chain which include alkyl, alkenyl, hydroxy alkyl, or keto alkyl radicals containing from 8 to 20 carbon atoms, from 0 to 10 ethylene oxide moieties and from 0 to 1 glyceryl moiety. Examples include: octadecyl methyl sulfoxide, 2-ketotridecyl methyl sulfoxide, 3,6,9,-trixaoctadecyl 2-hydroxyethyl sulfoxide, dodecyl methyl sulfoxide, oleyl 3-hydroxypropyl sulfoxide, tetradecyl methyl sulfoxide, 3-methoxytridecyl methyl sulfoxide, 3-hydroxytridecyl methyl sulfoxide, 3-hydroxy-4-dodecoxybutyl methyl sulfoxide.

Cationic surfactants useful in compositions of the present invention, particularly the conditioner compositions, contain amino or quaternary ammonium hydrophilic moieties which are positively charged when dissolved in the aqueous composition of the present invention. Cationic surfactants among those useful herein are disclosed in the following documents, M.C. Publishing Co., McCutcheon's, Detergents & Emulsifiers, (North American edition 1979); Schwartz, et al., Surface Active Agents, Their Chemistry and Technology, New

York: Interscience Publishers, 1949; US-A-3,155,591, Hilfer, issued November 3, 1964; US-A-3,929,678, Laughlin, et al., issued December 30, 1975; US-A-3,959,461, Bailey, et al., issued May 25, 1976; and US-A-4,387,090, Bolich, Jr., issued June 7, 1983. If included in the compositions of the present invention, the cationic surfactant is present at from 0.05% to 5%.

Among the quaternary ammonium-containing cationic surfactant materials useful herein are those of the general formula:

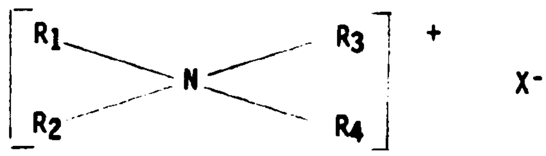

wherein $R_1$ - $R_4$ are independently an aliphatic group of from 1 to 22 carbon atoms, or an aromatic, alkoxy, polyoxyalkylene, alkylamido, hydroxyalkyl, aryl or alkylaryl group having from 12 to 22 carbon atoms; and X is an anion selected from halogen, acetate, phosphate, nitrate and alkylsulfate radicals. The aliphatic groups may contain, in addition to carbon and hydrogen atoms, ether linkages, and other groups such as amino groups.

Other quaternary ammonium salts useful herein have the formula:

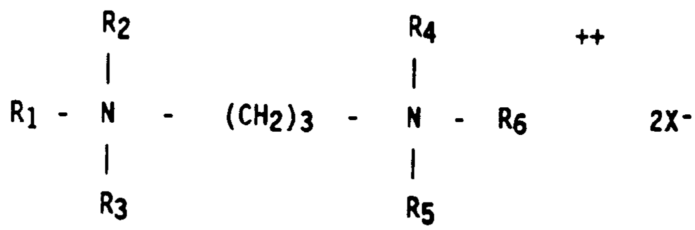

wherein $R_1$ is an aliphatic group having from 16 to 22 carbon atoms, $R_2$, $R_3$, $R_4$, $R_5$, and $R_6$ are selected from hydrogen and alkyl having from about 1 to about 4 carbon atoms, and X is an ion selected from halogen, acetate, phosphate, nitrate and alkyl sulfate radicals. Such quaternary ammonium salts include tallow propane diammonium dichloride.

Preferred quaternary ammonium salts include dialkyldimethylammonium chlorides, wherein the alkyl groups have from 12 to 22 carbon atoms and are derived from long-chain fatty acids, such as hydrogenated tallow fatty acid (tallow fatty acids yield quaternary compounds wherein $R_1$ and $R_2$ have predominately from 16 to 18 carbon atoms). Examples of quaternary ammonium salts useful in the present invention include ditallowdimethyl ammonium chloride, ditallowdimethyl ammonium methyl sulfate, dihexadecyl dimethyl ammonium chloride, di(hydrogenated tallow) dimethyl ammonium chloride, dioctadecyl dimethyl ammonium chloride, dieicosyl dimethyl ammonium chloride, didocosyl dimethyl ammonium chloride, di(hydrogenated tallow) dimethyl ammonium acetate, dihexadecyl dimethyl ammonium chloride, dihexadecyl dimethyl ammonium acetate, ditallow dipropyl ammonium phosphate, ditallow dimethyl ammonium nitrate, di(coconutalkyl) dimethyl ammonium chloride, and stearyl dimethyl benzyl ammonium chloride. Ditallow dimethyl ammonium chloride, dicetyl dimethyl ammonium chloride, stearyl dimethyl benzyl ammonium chloride and cetyl trimethyl ammonium chloride are preferred quaternary ammonium salts useful herein. Di-(hydrogenated tallow) dimethyl ammonium chloride is a particularly preferred quaternary ammonium salt.

Salts of primary, secondary and tertiary fatty amines are also preferred cationic surfactant materials. The alkyl groups of such amines preferably have from 12 to 22 carbon atoms, and may be substituted or unsubstituted. Secondary and tertiary amines are preferred, tertiary amines are particularly preferred. Such amines, useful herein, include stearamido propyl dimethyl amine, diethyl amino ethyl stearamide, dimethyl stearamine, dimethyl soyamine, soyamine, myristyl amine, tridecyl amine, ethyl stearylamine, N-tallowpropane diamine, ethoxylated (5 moles E.O.) stearylamine, dihydroxy ethyl stearylamine, and arachidylbehenylamine. Suitable amine salts include the halogen, acetate, phosphate, nitrate, citrate, lactate and alkyl sulfate salts. Such salts include stearylamine hydrochloride, soyamine chloride, stearylamine formate, N-tallowpropane diamine dichloride and stearamidopropyl dimethylamine citrate. Cationic amine surfactants included amont those useful in the present invention are disclosed in US-A-4,275,055, Nachtigal, et al., issued June 23, 1981,

Zwitterionic surfactants, useful in shampoos as well as conditioners, are exemplified by those which can be broadly described as derivatives of aliphatic quaternary ammonium, phosphonium, and sulfonium compounds, in which the aliphatic radicals can be straight or branched chain, and wherein one of the aliphatic substituents contains from 8 to 18 carbon atoms and one contains an anionic water-solubilizing group, e.g., carboxy, sulfonate, sulfate, phosphate, or phosphonate. A general formula for these compounds is:

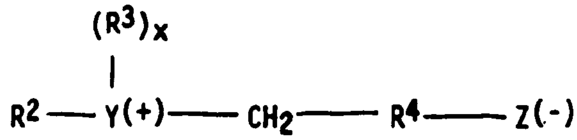

wherein $R^2$ contains an alkyl, alkenyl, or hydroxy alkyl radical of from 8 to 18 carbon atoms, from 0 to 10 ethylene oxide moieties and from 0 to 1 glyceryl moiety; Y is selected from the group consisting of nitrogen, phosphorus, and sulfur atoms; $R^3$ is an alkyl or monohydroxyalkyl group containing 1 to 3 carbon atoms; X is 1 when Y is a sulfur atom, and 2 when Y is a nitrogen or phosphorus atom; $R^4$ is an alkylene or hydroxyalkylene of from 1 to 4 carbon atoms and Z is a radical selected from the group consisting of carboxylate, sulfonate, sulfate, phosphonate, and phosphate groups.

Examples of such surfactants include:

4-[N,N-di(2-hydroxyethyl)-N-octadecylammonio]-butane-1-carboxylate;
5-[S-3-hydroxypropyl-S-hexadecylsulfonio]-3-hydroxypentane-1-sulfate;
3-[P,P-diethyl-P-3,6,9-trioxatetradexocylphosphonio]-2-hydroxy-propane-1-phosphate;
3-[N,N-dipropyl-N-3-dodecoxy-2-hydroxypropylammonio]-propane-1-phosphonate;
3-(N,N-dimethyl-N-hexadecylammonio)propane-1-sulfonate;
3-(N,N-dimethyl-N-hexadecylamnonio)-2-hydroxypropane-1-sulfonate;
4-[N,N-di(2-hydroxyethyl)-N-(2-hydroxydodecyl)ammonio]-butane-1-carboxylate;
3-[S-ethyl-S-(3-dodecoxy-2-hydroxypropyl)sulfonio]-propane-1-phosphate;
3-[P,P-dimethyl-P-dodecylphosphonio]-propane-1-phosphonate; and
5-[N,N-di(3-hydroxypropyl)-N-hexadecylamnonio]-2-hydroxy-pentane-1-sulfate.

Other zwitterionics such as betaines are also useful in the present invention. Examples of betaines useful herein include the high alkyl betaines, such as coco dimethyl carboxymethyl betaine, lauryl dimethyl carboxymethyl betaine, lauryl dimethyl alphacarboxyethyl betaine, cetyl dimethyl carboxymethyl betaine, lauryl bis-(2-hydroxyethyl) carboxymethyl betaine, stearyl bis-(2-hydroxypropyl) carboxymethyl betaine, oleyl dimethyl gamma-carboxypropyl betaine, and lauryl bis-(2-hydroxypropyl)alpha-carboxyethyl betaine. The sulfobetaines may be represented by coco dimethyl sulfopropyl betaine, stearyl dimethyl sulfopropyl betaine, lauryl dimethyl sulfoethyl betaine, lauryl bis-(2-hydroxyethyl) sulfopropyl betaine and the like; amidobetaines and amidosulfobetaines, wherein the $RCONH(CH_2)_3$ radical is attached to the nitrogen atom of the betaine are also useful in this invention.

Examples of amphoteric surfactants which can be used in the compositions of the present invention are those which are broadly described as derivatives of aliphatic secondary and tertiary amines in which the aliphatic radical can be straight or branched chain and wherein one of the aliphatic substituents contains from 8 to 18 carbon atoms and one contains an anionic water solubilizing group, e.g., carboxy, sulfonate, sulfate, phosphate, or phosphonate. Examples of compounds falling within this definition are sodium 3-dodecyl-aminopropionate, sodium 3-dodecylaminopropane sulfonate, N-alkyltaurines such as the one prepared by reacting dodecylamine with sodium isethionate according to the teaching of US-A-2,658,072, N-higher alkyl aspartic acids such as those produced according to the teaching of US-A-2,438,091, and the products sold under the trade name "Miranol" (RTM) and described in US-A-2,528,378.

The above-mentioned surfactants can be used alone or in combination in the hair care compositions of the present invention. The alkyl sulfates, ethoxylated alkyl sulfates and mixtures thereof are preferred for use herein.

The hair care compositions herein can contain a variety of other optional components suitable for rendering such compositions more cosmetically or aesthetically acceptable or to provide them with additional usage benefits. Such conventional optional ingredients are well-known to those skilled in the art, e.g., pearlescent aids, such as ethylene glycol distearate; preservatives, such as benzyl alcohol, methyl paraben, propyl paraben and imidazolidinyl urea; thickeners and viscosity modifiers, such as a diethanolamide of a long chain fatty acid (e.g., PEG 3 lauric diethanolamide), cocomonoethanol amide, dimethicone copolyols, guar gum, methyl cellulose, starches and starch derivatives; fatty alcohols, such as cetearyl alcohol; sodium chloride; sodium sulfate; polyvinyl alcohol; ethyl alcohol; pH adjusting agents, such as citric acid, sodium citrate, succinic acid, phosphoric acid, sodium hydroxide, and sodium carbonate; coloring agents, such as any of the FD&C or D&C dyes; hair oxidizing (bleaching) agents, such as hydrogen peroxide, perborate and persulfate salts; hair reducing agents, such as the thioglycolates; perfumes; sequestering agents, such as disodium ethylenediamine tetra-acetate; and polymer plasticizing agents, such as glycerin and propylene glycol. Such optional ingredients generally

are used individually at levels of from 0.01% to 10.0%, preferably from 0.05% to 5.0%, of the composition.

The pH of the present compositions should be between 3 and 9, preferably between 4 and 8.

As with all compositions, the present invention should not contain components which unduly interfere with the performance of the compositions.

The hair care compositions of the present invention can be made using conventional formulation and mixing techniques. Methods of making various types of hair care compositions are described more specifically in the following examples.

## Method of Use

The hair care compositions of the present invention are used in conventional ways to provide the hair conditioning/styling/hold benefits of the present invention. Such method of use depends upon the type of composition employed but generally involves application of an effective amount of the product to the hair, which may then be rinsed from the hair (as in the case of shampoos and some conditioning products) or allowed to remain on the hair (as in the case of spray, mousse, gel, and tonic products). By "effective amount" is meant an amount sufficient to provide the hair conditioning/styling/hold benefits desired considering the length and texture of the hair, and the type of product used. Preferably, the product is applied to wet or damp hair prior to drying and styling of the hair. After the compositions of the present invention are applied to the hair, the hair is dried and styled in the usual ways of the user.

The following examples further illustrate preferred embodiments within the scope of the present invention.

The following table defines the silicone copolymers used in the examples (weight ratios given refer to proportion added to reaction mix):

| | |
|---|---|
| Copolymer #1 | 10/70/20 acrylic acid/n-butylmethacrylate/silicone macromer S2, polymer molecular weight about 100,000 |
| Copolymer #2 | 10/70/20 dimethylaminoethyl methacrylate/isobutyl methacrylate/silicone macromer S2, polymer molecular weight about 400,000 |
| Copolymer #3 | 60/20/20 quaternized dimethylaminoethyl methacrylate/isobutyl methacrylate/silicone macromer S1, polymer molecular weight about 500,000 |
| Copolymer #4 | 40/40/20 acrylic acid/methyl methacrylate/silicone macromer S1, polymer molecular weight about 400,000 |
| Copolymer #5 | 10/70/20 acrylic acid/n-butyl methacrylate/silicone macromer S1, polymer molecular weight about 300,000 |
| Copolymer #6 | 25/65/10 acrylic acid/isopropyl methacrylate/silicone macromer S2, polymer molecular weight about 200,000 |
| Copolymer #7 | 60/25/15 N,N-dimethylacrylamide/methoxyethyl methacrylate/silicone macromer S1, polymer molecular weight about 200,000 |
| Copolymer #8 | 12/64/4/20 N,N-dimethylacrylamide/isobutyl methacrylate/2-ethylhexyl methacrylate/PDMS macromer S1, polymer molecular weight about 300,000 |
| Copolymer #9 | 30/40/10/20 dimethylaminoethyl methacrylate/isobutyl methacrylate/2-ethylhexyl methacrylate/PDMS macromer S1, polymer molecular weight about 300,000 |
| Copolymer #10 | 80/20 t-butylacrylate/PDMS macromer S2, polymer molecular weight about 150,000 |

Silicone macromer S1- has a molecular weight of 20,000 and is prepared in a manner similar to Example C-2c of US-A-4,728,571, Clemens, issued March 1, 1988.

Silicone macromer S2- has a molecular weight of 10,000 and is prepared in a manner similar to Example C-2b of US-A-4,728,571, Clemens, issued March 1, 1988.

## EXAMPLE I

The following is a hair spray composition representative of the present invention.

| Component | Weight % |
|---|---|
| Silicone Copolymer #4 | 2.00 |
| Ethanol | 72.90 |
| Perfume | 0.10 |
| Isobutane propellant | 25.00 |

This product is prepared by adding the silicone copolymer and perfume to the ethanol and mixing for several hours until all the polymer is dissolved. This "concentrate" is then placed in aerosol cans which are fitted with valves crimped under vacuum and then filled through the valve stem with isobutane dispensed by a pressure filler.

EXAMPLE II

The following is a shampoo composition representative of the present invention.

| Component | Weight % |
|---|---|
| Styling Agent | |
| Silicone Copolymer #2 | 1.00 |
| Chlorpropyl heptamethyl cyclotetrasiloxane | 3.00 |
| Premix | |
| Silicone gum | 0.50 |
| Dimethicone, 350 cs. fluid | 0.50 |
| Main Mix | |
| Ammonium lauryl sulfate | 11.00 |
| Cocamide MEA | 2.00 |
| Ethylene glycol distearate | 1.00 |
| Xanthan gum | 1.20 |
| Kathon (RTM) CG[1] | 0.04 |
| Citric acid to pH 4.5 | q.s. |
| Double reverse osmosis (DRO) $H_2O$ | q.s. |

[1] preservative commercially available from Rohm & Haas

The Styling Agent and Premix are blended separately in a conventional manner. The Main Mix is prepared by first dissolving the xanthan gum in the water with conventional mixing. The remaining Main Mix ingredients are added and the Main Mix is heated to 150°F with agitation for 1/2 hour. The Styling Agent and Premix are then added sequentially with ten minutes agitation between additions, and the entire mixture is stirred while the batch is cooled to room temperature. For varied particle size, the Styling Agent and Premix can be added at different times using either or both high shear mixing (high speed dispersator) or normal agitation.

EXAMPLE III

The following is a shampoo composition representative of the present invention.

| Component | Weight % |
|---|---|
| Ammonium lauryl sulfate | 7.00 |
| Ammonium laureth sulfate | 7.00 |
| Cocamide MEA | 2.50 |
| Silicone Copolymer #3 | 1.00 |
| Natrosol (RTM) 250H[1] | 1.00 |
| Glydant[2] (RTM) | 0.37 |
| DRO $H_2O$ | q.s. |

[1] hydroxyethyl cellulose commercially available from Aqualon Co.

[2] preservative commercially available from Glyco, Inc.

The shampoo is made by first dispersing the Natrosol (RTM) and silicone copolymer in the water for 1 hour with conventional agitation. The remaining ingredients are then added.

EXAMPLE IV

The following is a styling rinse composition representative of the present invention.

| Component | Weight % |
|---|---|
| Styling Agent Premix | |
| Silicone Copolymer #8 | 2.00 |
| Phenethylpentamethyl disiloxane | 6.00 |

| | |
|---|---|
| Octamethyl cyclotetrasiloxane | 3.00 |
| Xanthan Premix | |
| Xanthan gum | 0.25 |
| DRO $H_2O$ | 25.00 |
| Main Mix | |
| Dihydrogenated tallow-dimethylammonium chloride (DTDMAC) | 0.50 |
| EDTA, disodium salt | 0.10 |
| D.C. (RTM) 929[1] | 2.00 |
| Perfume | 0.10 |
| Poly Surf (RTM) C[2] | 0.75 |
| Locust bean gum | 0.75 |
| Kathon (RTM) CG[3] | 0.04 |
| DRO $H_2O$ | q.s. |

1 amodimethicone, commercially available from Dow Corning

2 hydrophobically- modified hydroxyethyl cellulose, commercially available from Aqualon Co.

3 preservative commercially available from Rohm and Haas

The Styling Agent and Xanthan Premixes are blended separately in a conventional manner. The Main Mix is prepared by adding all the ingredients together and heating with agitation to 95°C for 1/2 hour. As the batch is cooled, the Styling Agent and Xanthan Premixes are added at about 60°C with vigorous mixing. The batch is then cooled to ambient temperature.

EXAMPLE V

The following is a styling rinse composition representative of the present invention.

| Component | Weight % |
|---|---|
| Premix A | |
| Silicone Copolymer #3 | 2.00 |
| DRO $H_2O$ | 10.00 |
| Premix B | |
| Silicone Copolymer #4 | 2.00 |
| DRO $H_2O$ | 15.00 |
| NaOH solution (50%) | 0.20 |
| Main Mix | |
| Poly Surf (RTM) C[1] | 1.00 |

| | |
|---|---|
| Stearamide DEA | 0.50 |
| Ethanol | 10.00 |
| Perfume | 0.20 |
| DRO $H_2O$ | q.s. |

1 hydrophobically-modified hydroxyethyl cellulose, commercially available from Aqualon Co.

Both premixes are blended separately in a conventional manner. The Main Mix is prepared by adding all the ingredients together and heating to about 60°C with mixing. The premixes are then added to the Main Mix with agitation for 1/2 hour and the batch is cooled to ambient temperature. Either sodium hydroxide or citric acid, if necessary, is added to adjust composition pH to 6.5.

EXAMPLE VI

The following is a hair grooming tonic composition representative of the present invention.

| Component | Weight % |
|---|---|
| Silicone Copolymer #9 | 0.70 |
| Perfume | 0.10 |
| Ethanol | q.s. |

The composition is made by mixing the above components together in a conventional manner.

EXAMPLE VII

The following is a shampoo composition representative of the present invention.

| Component | Weight % |
|---|---|
| Ammonium laureth sulfate | 7.00 |
| Cocamido propyl betaine | 6.00 |
| Silicone Copolymer #6 | 2.00 |
| Ethanol | 10.00 |
| PEG 150 distearate | 2.00 |
| Glydant (RTM)[1] | 0.38 |
| Perfume | 1.00 |
| DRO $H_2O$ | q.s. |

1 preservative commercially available from Glyco, Inc.

The shampoo is prepared by combining the ammonium laureth sulfate (normally supplied as a 28% solution in water) and Silicone Copolymer and heating to 70°C for 1/2 hour with mixing. The remaining ingredients are added and mixed for an additional 1/2 hour. The batch is then cooled to ambient temperature. Composition pH is adjusted to 6.5 by the addition of citric acid or sodium hydroxide, if necessary.

EXAMPLE VIII

The following is a styling rinse composition representative of the present invention.

| Component | Weight % |
|---|---|
| Styling Agent | |
| Silicone Copolymer #5 | 3.00 |
| Phenylpentamethyl disiloxane | 9.00 |
| Premix | |
| Silicone Gum GE (RTM) SE76[1] | 0.50 |
| Decamethyl cyclopentasiloxane | 4.00 |
| Main Mix | |
| Poly Surf (RTM) C[2] | 0.60 |
| Locust bean gum | 0.50 |
| EDTA, disodium salt | 0.15 |
| DTDMAC | 0.65 |
| Glydant[3] | 0.40 |
| DRO $H_2O$ | q.s. |

[1] Commercially available from General Electric

[2] hydrophobically-modified hydroxyethyl cellulose commercially available from Aqualon Co.

[3] preservative commercially available from Glyco, Inc.

The Styling Agent and Premix are blended separately by conventional means. The Main Mix is prepared by adding all the ingredients and heating to 95°C for 1/2 hour with agitation. As the batch is cooled to 60°C, the Premix and Styling Agent mixes are added to the Main Mix with agitation and the batch is cooled to ambient temperature.

EXAMPLE IX

The following is a styling rinse composition representative of the present invention.

| Component | Weight % |
|---|---|
| Styling Agent | |

| | |
|---|---|
| Silicone Copolymer #10 | 3.00 |
| Octamethyl cyclotetrasiloxane | 9.00 |
| <u>Premix</u> | |
| Silicone Gum (RTM) GE SE76[1] | 0.50 |
| Decamethyl cyclopentosiloxane | 4.00 |
| <u>Main Mix</u> | |
| Poly Surf C (RTM)[2] | 1.25 |
| Stearamide DEA | 0.40 |
| DTDMAC | 0.50 |
| Kathon (RTM) CG[3] | 0.03 |
| Imidazole | 0.15 |
| Perfume | 0.10 |
| DRO $H_2O$ | q.s. |

1 Commercially available from General Electric

2 hydrophobically-modified hydroxyethyl cellulose commercially available from Aqualon Co.

3 preservative commercially available from Rohm & Haas

The Styling Agent and Premix are blended separately by conventional means. The Main Mix is prepared by adding all the ingredients and heating to 95°C for 1/2 hour with agitation. As the batch is cooled to 60°C, the Premix and Styling Agent mixes are added to the Main Mix with agitation and the batch is cooled to ambient temperature.

<u>EXAMPLE X</u>

The following is a cold-wave hair perm composition representative of the present invention.

| <u>Component</u> | <u>Weight %</u> |
|---|---|
| Thioglycolic acid | 5.00 |
| Monoethanolamine | 6.00 |
| Silicone Copolymer #3 | 1.50 |
| PEG 10 monostearate | 0.50 |
| DRO $H_2O$ | q.s. |

The composition is prepared by blending all the ingredients with agitation for 1/2 hour at 60°C and then cooling to ambient temperature.

<u>EXAMPLE XI</u>

The following is a hair conditioner composition representative of the present invention.

| Component | Weight % |
|---|---|
| Styling Agent Premix | |
| Silicone Copolymer #9 | 1.00 |
| Phenyl pentamethyl disiloxane | 4.00 |
| Silicone Premix | |
| Silicone gum, GE SE76[1] (RTM) | 0.30 |
| Octamethyl cyclotetrasiloxane | 1.70 |
| Main Mix | |
| Cetyl alcohol | 1.00 |
| Quaternium 18[2] (RTM) | 0.85 |
| Stearyl alcohol | 0.70 |
| Natrosol 250 MBR[3] (RTM) | 0.50 |
| Ceteareth-20 | 0.35 |
| Fragrance | 0.20 |
| Dimethicone copolyol | 0.20 |
| Citric acid | 0.13 |
| Methylchloroisothiazolinone, methylisothiazolinone | 0.04 |
| Sodium chloride | 0.01 |
| DRO $H_2O$ | q.s. |

[1] Commercially available from General Electric

[2] Ditallow quaternary ammonium compound, commercially available from Sherex

[3] hydroxyethyl cellulose material, commercially available from Aqualon Co.

The product is prepared by comixing all the Main Mix ingredients, heating to 60°C with mixing, and colloid milling down to 45°C. At this temperature, the two premixes are added separately with moderate agitation and the batch allowed to cool to ambient temperature.

EXAMPLE XII

The following is a styling gel composition representative of the present invention.

| Component | Weight % |
|---|---|
| Silicone Copolymer #7 | 2.00 |
| Carbopol 940[1] (RTM) | 0.75 |
| Triethanolamine | 1.00 |
| Dye solution | 0.05 |
| Perfume | 0.10 |
| Laureth-23 | 0.10 |
| DRO $H_2O$ | q.s. |

[1] cross-linked polyacrylic acid, commercially available from B. F. Goodrich

This batch is made by mixing the listed components together in a conventional manner.

EXAMPLE XIII

The following is a hair mousse composition representative of the present invention.

| Component | Weight % |
|---|---|
| Silicone Copolymer #7 | 3.00 |
| Ethanol | 15.00 |
| Cocamine oxide | 0.60 |
| D.C. 190[1] (RTM) | 0.20 |
| Cocamide DEA | 0.30 |
| Perfume | 0.10 |
| Isobutane | 7.00 |
| DRO $H_2O$ | q.s. |

[1] dimethicone copolyol, commercially available from Dow Corning

The composition is made by blending all of the ingredients except isobutane at ambient temperature until well mixed. Aluminum aerosol cans are then filled with 95 parts of this batch, affixed with a valve which is crimped into position, and lastly pressure filled with 5 parts isobutane.

EXAMPLE XIV

The following is a pump hair spray composition representative of the present invention.

| Component | weight % |
|---|---|
| Silicone Copolymer #1 | 2.50 |
| Dibutyl phthalate | 0.20 |
| Phenyldimethicone | 0.30 |

| | |
|---|---|
| Perfume | 0.05 |
| Aminomethyl propanol | 0.20 |
| Ethanol | q.s. |

This composition is made by mixing the listed components together in a conventional manner.

When the compositions defined in Examples I-XIV are applied to hair in the conventional manner, they provide effective hair conditioning and styling/hold benefits without leaving the hair with a sticky/stiff feel.

## Claims

1. A hair care composition characterized in that it comprises:
(a) from 0.1% by weight to 10.0% by weight of a silicone-containing copolymer having a molecular weight of from 10,000 to 1,000,000 comprising a component selected from: a lipophilic low polarity free radically polymerizable vinyl monomer (A), a hydrophilic polar monomer which is copolymerizable with A (B), and mixtures thereof; together with a silicone-containing macromer (C) having a weight average molecular weight of from 1,000 to 50,000 based on polydimethylsiloxane selected from

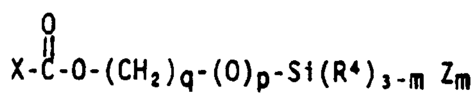

$X\text{-}Si(R^4)_{3\text{-}m}\,Z_m$

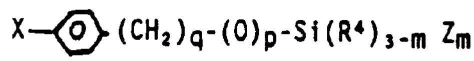

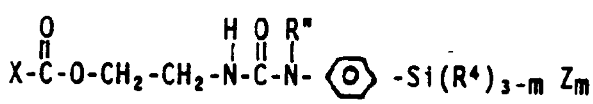

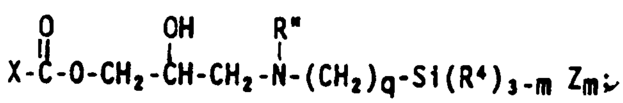 and

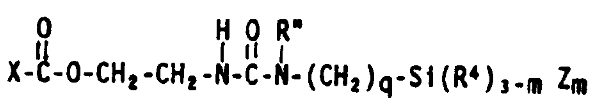

wherein m is 1, 2 or 3; p is 0 or 1; R" is alkyl or hydrogen; q is an integer from 2 to 6; s is an integer from 0 to 2; X is

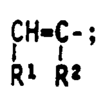

$R^1$ is hydrogen or -COOH; $R^2$ is hydrogen, methyl or $-CH_2COOH$; Z is

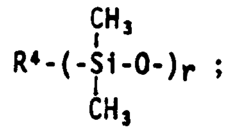

$R^4$ is alkyl, alkoxy, alkylamino, aryl or hydroxyl; and r is an integer from 5 to 700;
and wherein the silicone-containing copolymer comprises from 0% to 98% by weight monomer A, from 0% to 98% by weight monomer B, and from 0.1% by weight to 50% by weight monomer C; and
(b) from 0.5% by weight to 99.5% by weight of a carrier suitable for application to hair.

**2.** A hair care composition according to Claim 1 characterized in that the silicone-containing copolymer comprises from 5% by weight to 98% by weight monomer A, from 7.5% by weight to 80% by weight monomer B, and from 0.1% by weight to 50% by weight monomer C.

**3.** A hair care composition according to Claim 1 or 2 characterized in that monomer A is selected from acrylic acid esters of $C_1$-$C_{18}$ alcohols, methacrylic acid esters of $C_1$-$C_{18}$ alcohols, styrene, polystyrene macromer, vinyl acetate, vinyl chloride, vinyl propionate, vinylidene chloride, alpha-methylstyrene, t-butylstyrene, butadiene, cyclohexadiene, ethylene, propylene, vinyl toluene, and mixtures thereof; and is preferably selected from n-butylmethacrylate, isobutylmethacrylate, 2-ethylhexylmethacrylate, methylmethacrylate, t-butylacrylate, t-butylmethacrylate, and mixtures thereof.

**4.** A hair care composition according to any of Claims 1-3 characterized in that monomer B is selected from acrylic acid, methacrylic acid, N,N-dimethylacrylamide, dimethylaminoethyl methacrylate, quaternized dimethylaminoethyl methacrylate, methacrylamide, N-t-butyl acrylamide, maleic acid, maleic anhydride, half esters of maleic anhydride, crotonic acid, itaconic acid, acrylamide, acrylate alcohols, hydroxyethyl methacrylate, diallyldimethyl ammonium chloride, vinyl pyrrolidone, vinyl ethers, maleimides, vinyl pyridine, vinyl imidazole, styrene sulfonate, allyl alcohol, vinyl alcohol, vinyl caprolactam, and mixtures thereof; and is preferably selected from acrylic acid, N,N-dimethylacrylamide, dimethylaminoethyl methacrylate, quaternized dimethylaminoethyl methacrylate, vinyl pyrrolidone, and mixtures thereof.

**5.** A hair care composition according to any of Claims 1-4 characterized in that monomer C has the formula

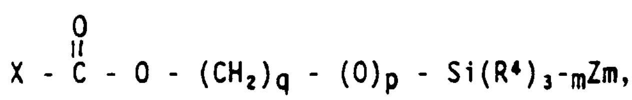

wherein p = 0 and q = 3, m is 1, r is 250, $R^4$ is alkyl, $R^1$ is hydrogen, and $R^2$ is methyl.

**6.** A hair care composition according to any of Claims 1-5 characterized in that the silicone-containing copolymer is selected from:
acrylic acid/n-butylmethacrylate/polydimethylsiloxane (PDMS) macromer - 20,000 mw (10/70/20);
N,N-dimethylacrylamide/isobutyl methacrylate/PDMS macromer 20,000 mw (20/60/20);
dimethylaminoethyl methacrylate/isobutyl methacrylate/2-ethylhexyl methacrylate/PDMS macromer - 20,000 mw (25/40/15/20);
dimethylaminoethyl methacrylate/isobutyl methacrylate/PDMS macromer - 20,000 mw (10/70/20);
quaternized dimethylaminoethyl methacrylate/isobutyl methacrylate/PDMS macromer - 20,000 mw (40/40/20);
acrylic acid/methyl methacrylate/PDMS macromer - 20,000 mw (40/40/20);
acrylic acid/isopropyl methacrylate/PDMS macromer - 20,000 mw (25/65/10);
N,N-dimethylacrylamide/methoxyethyl methacrylate/PDMS macromer - 20,000 mw (60/25/15);
dimethylacrylamide/PDMS macromer - 20,000 mw (80/20);
and mixtures thereof.

**7.** A hair care composition according to any of Claims 1-6 characterized in that it is in the form of a shampoo which additionally comprises from 10% by weight to 30% by weight of a synthetic surfactant, which is preferably selected from alkyl sulfates, ethoxylated alkyl sulfates, and mixtures thereof.

8. A hair care composition according to any of Claims 1-6 characterized in that it is in the form of a conditioner in which the carrier comprises from 0.1% by weight to 20.0% by weight of a lipid vehicle material, preferably selected from cetyl alcohol, stearyl alcohol, cetyl palmitate, glyceryl monostearate, and mixtures thereof; and from 0.05% by weight to 5.0% by weight of a cationic surfactant, preferably a quaternary ammonium surfactant.

9. A hair care composition according to any of Claims 1-6 characterized in that it is in a form selected from hair sprays, mousses, hair tonics and gels.

10. A method of conditioning and styling hair characterized in that it comprises applying to the hair an effective amount of the composition according to any of Claims 1-9.

## Patentansprüche

1. Haarpflegezusammensetzung, dadurch gekennzeichnet, daß sie
(a) 0,1 Gew.-% bis 10,0 Gew.-% eines Silikon enthaltenden Copolymers mit einem Molekulargewicht von 10.000 bis 1,000.000, welches eine Komponente umfaßt, die unter: einem lipophilen frei-radikalisch polymerisierbaren Vinylmonomer geringer Polarität (A), einem hydrophilen polaren Monomer, welches mit A copolymerisierbar ist, (B), und Gemischen hievon ausgewählt ist; gemeinsam mit einem Silikon enthaltenden Makromer (C), welches ein Gewichtsmittel-Molekulargewicht von 1.000 bis 50.000 besitzt, welches auf einem Polydimethylsiloxan beruht, das unter

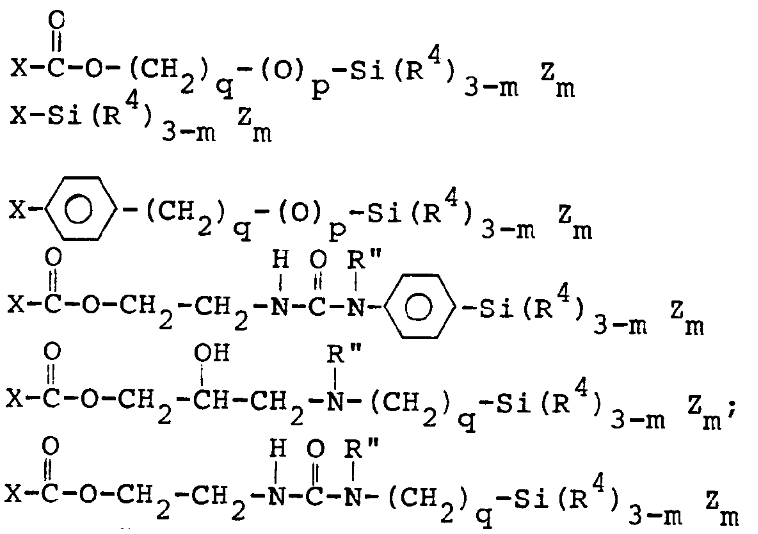
und

ausgewählt ist, worin m 1, 2 oder 3 bedeutet; p 0 oder 1 ist; R" für Alkyl oder Wasserstoff steht; q eine ganze Zahl von 2 bis 6 ist; s eine ganze Zahl von 0 bis 2 darstellt; X für

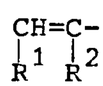

steht;
$R^1$ Wasserstoff oder -COOH bedeutet; $R^2$ Wasserstoff, Methyl oder $-CH_2COOH$ ist; Z für

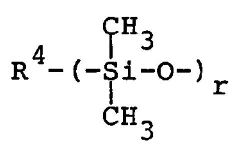

steht;

$R^4$ Alkyl, Alkoxy, Alkylamino, Aryl oder Hydroxyl bedeutet; und r eine ganze Zahl von 5 bis 700 ist; und worin das Silikon enthaltende Copolymer 0 Gew.-% bis 98 Gew.-% an Monomer A, 0 Gew.-% bis 98 Gew.-% an Monomer B und 0,1 Gew.-% bis 50 Gew.-% an Monomer C enthält; und
(b) 0,5 Gew.-% bis 99,5 Gew.-% eines Trägers umfaßt, welcher zur Aufbringung auf Haar geeignet ist.

2. Haarpflegezusammensetzung nach Anspruch 1, dadurch gekennzeichnet, daß das Silikon enthaltende Copolymer 5 Gew.-% bis 98 Gew.-% an Monomer A, 7,5 Gew.-% bis 80 Gew.-% an Monomer B und 0,1 Gew.-% bis 50 Gew.-% an Monomer C umfaßt.

3. Haarpflegezusammensetzung nach Anspruch 1 oder 2, dadurch gekennzeichnet, daß das Monomer A unter Acrylsäureestern von $C_1$-$C_{18}$Alkoholen, Methacrylsäureestern von $C_1$-$C_{18}$-Alkoholen, Styrol, Polystyrolmakromer, Vinylacetat, Vinylchlorid, Vinylpropionat, Vinylidenchlorid, alpha-Methylstyrol, tert.Butylstyrol, Butadien, Cyclohexadien, Ethylen, Propylen, Vinyltoluol, und Gemischen hievon ausgewählt ist; und vorzugsweise unter n-Butylmethacrylat, Isobutylmethacrylat, 2-Ethylhexylmethacrylat, Methylmethacrylat, tert.Butylacrylat, tert.Butylmethacrylat, und Gemischen hievon ausgewählt ist.

4. Haarpflegezusammensetzung nach einem der Ansprüche 1 bis 3, dadurch gekennzeichnet, daß das Monomer B unter Acrylsäure, Methacrylsäure, N,N-Dimethylacrylamid, Dimethylaminoethylmethacrylat, quaternisiertem Dimethylaminoethylmethacrylat, Methacrylamid, N-tert.Butylacrylamid, Maleinsäure, Maleinsäureanhydrid, Halbestern von Maleinsäureanhydrid, Crotonsäure, Itaconsäure, Acrylamid, Acrylatalkoholen, Hydroxyethylmethacrylat, Diallyldimethylammoniumchlorid, Vinylpyrrolidon, Vinylethern, Maleinimiden, Vinylpyridin, Vinylimidazol, Styrolsulfonat, Allylalkohol, Vinylalkohol, Vinylcaprolactam, und Gemischen hievon ausgewählt ist; und vorzugsweise unter Acrylsäure, N,N-Dimethylacrylamid, Dimethylaminoethylmethacrylat, quaternisiertem Dimethylaminoethylmethacrylat, Vinylpyrrolidon, und Gemischen hievon ausgewählt ist.

5. Haarpflegezusammensetzung nach einem der Ansprüche 1 bis 4, dadurch gekennzeichnet, daß das Monomer C die Formel

$$X - \overset{O}{\overset{\|}{C}} - O - (CH_2)_q - (O)_p - Si(R^4)_{3-m}Z_m$$

besitzt, worin p für 0 steht und q 3 ist, m 1 bedeutet, r 250 beträgt, $R^4$ für Alkyl steht, $R^1$ Wasserstoff bedeutet und $R^2$ Methyl darstellt.

6. Haarpflegezusammensetzung nach einem der Ansprüche 1 bis 5, dadurch gekennzeichnet, daß das Silikon enthaltende Copolymer unter:
Acrylsäure/n-Butylmethacrylat/Polydimethylsiloxan (PDMS)-Makromer - MG 20.000 (10/70/20);
N,N-Dimethylacrylamid/Isobutylmethacrylat/PDMS-Makromer - MG 20.000 (20/60/20);
Dimethylaminoethylmethacrylat/Isobutylmethacrylat/2-Ethylhexylmethacrylat/PDMS-Makromer - MG 20.000 (25/40/15/20);
Dimethylaminoethylmethacrylat/Isobutylmethacrylat/PDMS-Makromer - MG 20.000 (10/70/20);
quaternisiertem Dimethylaminoethylmethacrylat/Isobutylmethacrylat/PDMS-Makromer - MG 20.000 (40/40/20);
Acrylsäure/Methylmethacrylat/PDMS-Makromer - MG 20.000 (40/40/20);
Acrylsäure/Isopropylmethacrylat/PDMS-Makromer - MG 20.000 (25/65/10);
N,N-Dimethylacrylamid/Methoxyethylmethacrylat/PDMS-Makromer - MG 20.000 (60/25/15);
Dimethylacrylamid/PDMS-Makromer - MG 20.000 (80/20)
und Gemischen hievon ausgewählt ist.

7. Haarpflegezusammensetzung nach einem der Ansprüche 1 bis 6, dadurch gekennzeichnet, daß sie in der Form eines Shampoos vorliegt, welches zusätzlich 10 Gew.-% bis 30 Gew.-% eines synthetischen grenzflächenaktiven Mittels enthält, welches vorzugsweise unter Alkylsulfaten, ethoxylierten Alkylsulfaten und Gemischen hievon ausgewählt ist.

8. Haarpflegezusammensetzung nach einem der Ansprüche 1 bis 6, dadurch gekennzeichnet, daß sie in der Form eines Konditionierungsmittels vorliegt, worin der Träger 0,1 Gew.-% bis 20,0 Gew.-% eines Lipidträgermaterials, welches vorzugsweise unter Cetylalkohol, Stearylalkohol, Cetylpalmitat,

Glycerylmonostearat und Gemischen hievon ausgewählt ist; und 0,05 Gew.-% bis 5,0 Gew.-% eines kationischen grenzflächenaktiven Mittels, vorzugsweise ein quaternäres Ammonium-grenzflächenaktives Mittel, enthält.

9. Haarpflegezusammensetzung nach einem der Ansprüche 1 bis 6, dadurch gekennzeichnet, daß sie in einer Form vorliegt, die unter Haarsprays, -schäumen, Haartonika und -gelen ausgewählt ist.

10. Verfahren zum Haarkonditionieren und -formen, dadurch gekennzeichnet, daß es das Aufbringen einer wirksamen Menge der Zusammensetzung nach einem der Ansprüche 1 bis 9 auf Haar umfaßt.

## Revendications

1. Composition de soin de cheveux, caractérisée en ce qu'elle comprend:
(a) de 0,1% en poids à 10,0% en poids d'un copolymère contenant une silicone, ayant un poids moléculaire de 10.000 à 1.000.000, comprenant un composant choisi parmi: un monomère vinylique lipophile de faible polarité, polymérisable par polymérisation radicalaire (A), un monomère polaire hydrophile copolymérisable avec A (B), et leurs mélanges, ainsi qu'un macromère contenant une silicone (C) ayant un poids moléculaire moyen en poids de 1.000 à 50.000 d'un polydiméthylsiloxane choisi parmi

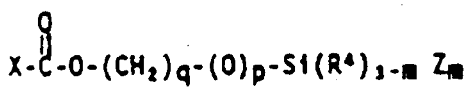

$$X\text{-}Si(R^4)_{3-m}\,Z_m$$

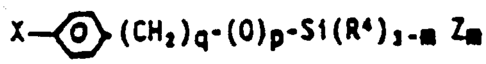

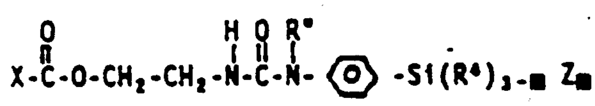

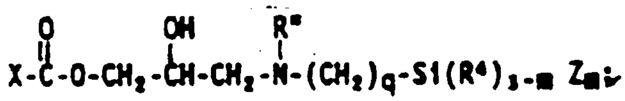 et

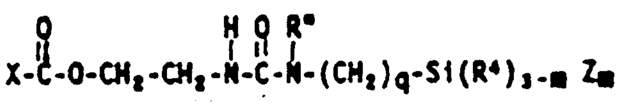

où m est égal à 1, 2 ou 3; p est égal à 0 ou 1; R" est un groupe alkyle ou un atome d'hydrogène; q est un nombre entier de 2 à 6; s est un nombre entier de 0 à 2; X est

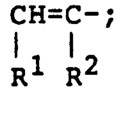

$R^1$ est un atome d'hydrogène ou un groupe -COOH; $R^2$ est un atome d'hydrogène, un groupe méthyle ou $-CH_2COOH$; Z est

$$R^4-(-\overset{CH_3}{\underset{CH_3}{\overset{|}{\underset{|}{Si}}}}-O-)_r \;;$$

$R^4$ est un groupe alkyle, alcoxy, alkylamino, aryle ou hydroxyle; et r est un nombre entier de 5 à 700; et où le copolymère contenant une silicone comprend de 0% à 98% en poids de monomère A, de 0% à 98% en poids de monomère B et de 0,1% en poids à 50% en poids de monomère C; et (b) de 0,5% en poids à 99,5% en poids d'un véhicule convenant à l'application sur les cheveux.

2. Composition de soin des cheveux selon la revendication 1, caractérisée en ce que le copolymère contenant une silicone comprend de 5% en poids à 98% en poids de monomère A, de 7,5% en poids à 80% en poids de monomère B et de 0,1% en poids à 50% en poids de monomère C.

3. Composition de soin des cheveux selon la revendication 1 ou 2, caractérisée en ce que le monomère A est choisi parmi les esters d'acide acrylique et d'alcools en $C_1$-$C_{18}$, les esters d'acide méthacrylique et d'alcools en $C_1$-$C_{18}$, le styrène, un macromère polystyrène, l'acétate de vinyle, le chlorure de vinyle, le propionate de vinyle, le chlorure de vinylidène, l'$\alpha$-méthylstyrène, le t-butylstyrène, le butadiène, le cyclohexadiène, l'éthylène, le propylène, le vinyltoluène, et leurs mélanges; et est de préférence choisi parmi le méthacrylate de n-butyle, le méthacrylate d'isobutyle, le méthacrylate de 2-éthylhexyle, le méthacrylate de méthyle, l'acrylate de t-butyle, le méthacrylate de t-butyle, et leurs mélanges.

4. Composition de soin des cheveux selon l'une quelconque des revendications 1-3, caractérisée en ce que le monomère B est choisi parmi l'acide acrylique, l'acide méthacrylique, le N,N-diméthylacrylamide, le méthacrylate de diméthylaminoéthyle, le méthacrylate de diméthylaminoéthyle quaternisé, le méthacrylamide, le N-t-butylacrylamide, l'acide maléique, les demi-esters d'anhydride maléique, l'acide crotonique, l'acide itaconique, l'acrylamide, les alcools d'acrylate, le méthacrylate d'hydroxyéthyle, le chlorure de diallyldiméthylammonium, la vinylpyrrolidone, les éthers vinyliques, les maléimides, la vinylpyridine, le vinylimidazole, les styrène-sulfonate, l'alcool allylique, l'alcool vinylique, le vinylcaprolactame, et leurs mélanges; et est de préférence choisi parmi l'acide acrylique, le N,N-diméthylacrylamide, le méthacrylate de diméthylaminoéthyle, le méthacrylate de diméthylaminoéthyle quaternisé, la vinylpyrrolidone, et leurs mélanges.

5. Composition de soin des cheveux selon l'une quelconque des revendications 1-4, caractérisée en ce que le monomère C a pour formule:

$$X - \overset{O}{\overset{\|}{C}} - O - (CH_2)_q - (O)_p - Si(R^4)_{3-m}\, Z_m$$

dans laquelle p = 0 et q = 3, m est égal à 1, r est égal à 250, $R^4$ est un groupe alkyle, $R^1$ est un atome d'hydrogène et $R^2$ est un groupe méthyle.

6. Composition de soin de cheveux selon l'une quelconque des revendications 1-5, caractérisée en ce que le copolymère contenant une silicone est choisi parmi les séquences suivantes:
acide acrylique/méthacrylate de n-butyle/macromère polydiméthylsiloxane (PDMS) - P.M. 20.000 510/70/20)
N,N-diméthylacrylamide/méthacrylate d'isobutyle/macromère PDMS - P.M. 20.000 (20/60/20);
méthacrylate de diméthylaminoéthyle/méthacrylate d'isobutyle/méthacrylate de 2-éthylhexyle/macromère PDMS - P.M. 20.000 (25/40/15/20);
méthacrylate de diméthylaminoéthyle/méthacrylate d'isobutyle/macromère PDMS - P.M. 20.000 (10/70/20);
méthacrylate de diméthylaminoéthyle quaternisé/méthacrylate d'isobutyle/macromère PDMS - P.M. 20.000 (40/40/20);
acide acrylique/méthacrylate de méthyle/macromère PDMS - P.M. 20.000 (40/40/20);
acide acrylique/méthacrylate d'isopropyle/macromère PDMS P.M. 20.000 (25/65/10);

N,N-diméthylacrylamide/méthacrylate de méthoxyéthyle/macromère PDMS - P.M. 20.000 (60/25/15); diméthylacrylamide/macromère PDMS - P.M. 20.000 (80/20); et leurs mélanges.

**7.** Composition de soin des cheveux selon l'une quelconque des revendications 1-6, caractérisée en ce qu'elle se trouve sous la forme d'un shampooing qui comprend, en outre, de 10% en poids à 30% en poids d'un tensioactif synthétique, qui est de préférence choisi parmi les alkylsulfates, les alkylsulfates éthoxylés, et leurs mélanges.

**8.** Composition de soin des cheveux selon l'une quelconque des revendications 1-6, caractérisée en ce qu'elle se trouve sous la forme d'un conditionneur dans lequel le véhicule comprend de 0,1% en poids à 20,0% en poids d'un véhicule, choisi de préférence parmi l'alcool cétylique, l'alcool stéarylique, le palmitate de cétyle, le monostéarate de glycéryle, et leurs mélanges; et de 0,05% en poids à 5,0% en poids d'un tensioactif cationique, de préférence d'un tensioactif d'ammonium quaternaire.

**9.** Composition de soin des cheveux selon l'une quelconque des revendications 1-6, caractérisée en ce qu'elle se trouve sous une forme choisie parmi les laques, mousses, toniques et gels pour les cheveux.

**10.** Procédé de conditionnement et de coiffage des cheveux, caractérisé en ce qu'il comprend l'application sur les cheveux d'une quantité efficace de la composition selon l'une quelconque des revendications 1-9.